# EUROPEAN PATENT SPECIFICATION

(11) **EP 3 813 711 B1**
(45) Date of publication and mention of the grant of the patent: **11.05.2022**
(21) Application number: 19733704.1
(22) Date of filing: 19.06.2019
(51) Int. Cl.: A61B 34/20, A61B 34/10, A61B 17/00, A61B 90/00

(54) **OPTIMAL IMAGING POINT OF VIEW BASED ON INTERVENTION INSTRUMENT LOADING**
OPTIMALE BILDGEBUNGSPERSPEKTIVE BASIEREND AUF EINER EINGRIFFSINSTRUMENTENBELASTUNG
POINT DE VUE D'IMAGERIE OPTIMAL BASÉ SUR LE CHARGEMENT D'UN INSTRUMENT D'INTERVENTION

(30) Priority: 26.06.2018 US 201862690127 P
(43) Date of publication of application: 05.05.2021
(73) Proprietor: Koninklijke Philips N.V., 5656 AG Eindhoven (NL)
(72) Inventor: BALICKI, Marcin, Arkadiusz, 5656 AE Eindhoven (NL)
(74) Representative: Philips Intellectual Property & Standards
(86) International application number: PCT/EP2019/066176
(87) International publication number: WO 2020/002078

(56) References cited:
- WO-A1-2017/042823
- WO-A2-2007/141784
- US-A1- 2010 169 815
- US-A1- 2015 100 066
- US-A1- 2018 132 839

## Description

### FIELD OF THE INVENTION

The present disclosure generally relates to an imaging point of view during an interventional procedure. The present disclosure specifically relates to novel and inventive optimal imaging point of view based on forces and torques applied to an intervention instrument.

### BACKGROUND OF THE INVENTION

In image guided Surgery, surgeons transfer a pre-operative surgical treatment plan from a three-dimensional ("3D") virtual model directly onto the patient's anatomy using a guide positioning system that tracks the patient, the instrument, and performs a registration to bring the surgical plan into the patient coordinate system. In many procedures passive or robotic instrument guides are used to provide stability and precision in positioning and aligning the instrument with the target using the guide positioning system feedback. In particular, navigation assistance is required in Minimally Invasive Surgery (MIS), where access to the intervention site is minimized to only accept special instruments (often tubes, or rods, or needles). MIS improves healing time, reduce complications due to infections, reduces blood loss, improves cosmetic results, reduces costs, etc. A guide positioning system enables the surgeon to overcome visual occlusions and geometric access limitations due to this MIS approach, and provides high degree of accuracy in placement of instruments relative to the anatomy.

For example, MIS Spine fusion surgery is a procedure that is enabled by surgical navigation. Traditional approaches for placement of screws requires extensive manipulation and removal of muscle and other tissues to access the surface of the spine, in comparison, MIS allows percutaneous approach for pedicle screw fixation. Specifically, the surgeon plans placement of pedicle screws on a computed-tomography ("CT") imaged 3D model of the patient and places instrumentation in the pedicle of the vertebrae through the skin without seeing the pedicle itself. The instrument guidance system (e.g., optical tracker based, or robotics positioner) tracks the patient and the instrument to provide visual guidance to the surgeon on the location of the target pedicle, centimeters below the skin. A few 2D or 3D X-Ray images, are often taken early in the procedure to register the preparative 3D model and surgical plan to the patient with tracked instruments and fiducial markers. The positioning on of the instrument may be done in freehand fashion or with a manually or robotically positioned instrument guides. The positioning arms and robotically placed guides provide a stable trajectory for the instrument (e.g., pedicle awl, k-wire) to be inserted into the patient through the skin, fat, muscle and eventually layers of bone. Robotic guide automatically places the guide into planned position, thereby reducing the reliance on hand-eye coordination to interpret the location of the target trajectory relative to current instrument position. Some protocols include verification X-Rays of instruments (i.e., needle-like k-wire) placed in the patient before the screws are inserted in the pedicle. Surgeon will attempt to reinsert the k-wire if misalignment is visible in the X-ray projections. After the screws are fixed, additional verification images are taken before the incisions are closed.

Similar processes are found in many Image Guided Therapies such as, for example, biopsies or ablations where needle like devices are inserted into the patient percutaneously to reach a target mass inside the patient.

As an example, WO 2007/141784 A2 discloses a robotic system for steering a flexible needle during insertion into soft tissue using imaging. A control system calculates a needle tip trajectory that hits the desired target while avoiding potentially dangerous obstacles en route. Using an inverse kinematics algorithm, the manoeuvres required of the needle base to cause the tip to follow this trajectory are calculated. In particular, the forward and inverse kinematics of the needle are solved analytically, enabling both path planning and correction in real-time.

However, even with navigation and robotic guidance, precise positioning is prone to errors especially for long thin instruments (needles, drills, etc.,) used through small incisions. This is due to the instrument or tissue displacement by the forces exerted on the instrument by the tissue: skin, muscle, and bone that comes in contact with the instrument, as well as the forces applied by the surgeon. Even with a rigid robotic guide systems (e.g., Mazor X), these forces may deflect the target tissue, the robotic manipulator, and the instrument itself, resulting in a deviation from the intended target path leading to surgical errors. This deviation is not always easily detected or corrected immediately.

In many cases the trajectory is verified with additional imaging (X-ray) right before the interventional step is complete (e.g., k-wire placement for verification before the pedicle screw is implanted), and in many cases this step is performed at the end of the procedure to verify that all the planned interventions have been executed properly, in which case corrections are not easily remedied. Therefore, the surgeon would benefit from knowing that a potential positional error is possible due to loading of the instrument, during or immediately after the placement of the instrument. In addition, the direction of this load may indicate the direction of potential positional or angulation errors. A method for assisting the surgeon in imaging the patient to assess any positional error undetected by navigation/tracking systems is therefore needed.

### SUMMARY OF THE INVENTION

The present invention is defined by the features of the independent claim. Preferred embodiments are given in the dependent claims. Any methods described herein do not form part of the claimed invention.

To improve upon positional errors undetected by navigation/tracking systems, the inventions of the present disclosure provides optimal imaging POV intervention systems for detecting forces/torques exerted on an intervention instrument (e.g., needles, drills, screws, etc.) and/or an instrument guide (e.g., handheld, passive positioning arm, or a fully actuated robotic positioner) during the intervention. Any potential for a positioning error of the intervention instrument due to the detected forces/torques may be reported to the surgeon whereby the surgeon may adjust the POV of the medical imaging system for an optimal view of the potential positioning error or whereby controllers of the present disclosure may autonomously adjust the POV of the medical imaging for an optimal view of the potential positioning error. The systems and methods of the present disclosure may suggest point-of-view for verification imaging (e.g., imaging position and axis angles in case of a C-Arm) derived from the direction and/or magnitude of the detected forces/torques and position and orientation of the intervention instrument relative to the anatomy and/or treatment plan.

One embodiment of the inventions of the present disclosure is an optimal imaging POV intervention system employs an intervention instrument, an instrument guide, one or more force/torque sensors and an optimal imaging POV controller. In operation, the instrument guide establishes a planned trajectory of the interventional instrument, and the force/torque sensor(s) sense a force and/or a torque exerted the intervention instrument and/or the instrument guide. The optimal imaging POV controller controls a determination of an optimal imaging POV of the intervention instrument by deriving an imaging axis of the intervention instrument from a measurement of the force and/or the torque exerted against the intervention instrument and/or the instrument guide as sensed by the force/torque sensor(s).

A second embodiment of the inventions of the present disclosure an optimal imaging POV intervention system involving (1) establishing, by an instrument guide, a planned trajectory of an interventional instrument, (2) sensing, by one or more force/torque sensors, a force and/or a torque exerted against the intervention instrument and/or the instrument guide when the intervention instrument is positioned within the instrument guide, and (3) controlling, by an optimal imaging POV controller, a determination of an optimal imaging POV of the intervention instrument by deriving an imaging axis of the intervention instrument from a measurement of the force and/or the torque exerted against the intervention instrument and/or the instrument guide as sensed by the force/torque sensor(s).

Generally, the optimal POV is based on the maximum force and/or torque (with empirical determined relative weights) over the course of the task (e.g., application of the instrument on the body.)

Alternatively, a temporal filter may be employed that compares the average force/torque over periods in time. In this mode, the optimal imaging POV controller suggests multiple views that correspond to (1) a short event with high force/torque (such as an impulse from a hammer), (2) a view associated with a constant force/torque measured over a period of s seconds, or (3) average force/torque over a period of s seconds.

In addition, optimal imaging POV controller may link force-torque to the position of the tool in the body and consider a surgical plan that includes the expected forces and torques on the instrument during the procedure. The deviations from this plan are used to generate the POV as described above.

Other optimal POVs can be associated with maximum force and torque at a particular area of interest in the body, such as depth (1cm below skin) or proximity to tissue (on bone).

In the case where the instrument is able to characterize the tissue (e.g., using spectral sensing), prioritization to the potential POV selection (from force/torque measurements) can be ordered by the tissue type and position within the body or relative to the target or instrument.

The optimal imaging POV controller may also provide an interface for the surgeon to select the POV derived from the force/torque at the surgeon-selected time during the task or surgeon-selected pre-recorded position of the instrument during the task.

For purposes of describing and claiming the inventions of the present disclosure:
(1) terms of the art of the present disclosure including, but not limited to, "interventional procedure", "intervention instrument", "instrument guide", "intervention imaging system", "position tracking system", "guide positioning system", "force sensor", "torque sensor", "force measurements" and "torque measurements" are to be broadly interpreted as known in the art of the present disclosure and exemplary described in the present disclosure;
(2) more particularly, the term "interventional procedure" broadly encompasses all interventional procedures, as known in the art of the present disclosure or hereinafter conceived, for an imaging, a diagnosis and/or a treatment of a patient anatomy;
(3) more particularly, the term "force/torque sensor" broadly encompasses all force sensors, torque sensors or combination thereof, as known in the art of the present disclosure or hereinafter conceived, for sensing a force and/or a torque exerted, directly or indirectly, against the intervention instrument and the instrument guide;
(4) the term "controller" broadly encompasses all structural configurations, as understood in the art of the present disclosure and as exemplary described in the present disclosure, of an application specific main board or an application specific integrated circuit for controlling an application of various inventive principles of the present disclosure as subsequently described in the present disclosure. The structural configuration of the controller may include, but is not limited to, processor(s), computer-usable/computer readable storage medium(s), an operating system, application module(s), peripheral device controller(s), slot(s) and port(s). A controller may be housed within or linked to a workstation. Examples of a "workstation" include, but are not limited to, an assembly of one or more computing devices, a display/monitor, and one or more input devices (e.g., a keyboard, joysticks and mouse) in the form of a standalone computing system, a client computer of a server system, a desktop, a laptop or a tablet;
(5) the descriptive labels for controllers described and claimed herein facilitate a distinction between controllers as described and claimed herein without specifying or implying any additional limitation to the term "controller";
(6) the term "application module" broadly encompasses an application incorporated within or accessible by a controller consisting of an electronic circuit (e.g., electronic components and/or hardware) and/or an executable program (e.g., executable software stored on non-transitory computer readable medium(s) and/or firmware) for executing a specific application;
(7) the descriptive labels for application modules described and claimed herein facilitate a distinction between application modules as described and claimed herein without specifying or implying any additional limitation to the term "controller";
(8) the terms "signal", "data" and "command" broadly encompasses all forms of a detectable physical quantity or impulse (e.g., voltage, current, or magnetic field strength) as understood in the art of the present disclosure and as exemplary described in the present disclosure for transmitting information and/or instructions in support of applying various inventive principles of the present disclosure as subsequently described in the present disclosure. Signal/data/command communication various components of the present disclosure may involve any communication method as known in the art of the present disclosure including, but not limited to, signal/data/command transmission/reception over any type of wired or wireless datalink and a reading of signal/data/commands uploaded to a computer-usable/computer readable storage medium; and
(9) the descriptive labels for signals/data/commands as described and claimed herein facilitate a distinction between signals/data/commands as described and claimed herein without specifying or implying any additional limitation to the terms "signal", "data" and "command".

The foregoing embodiments and other embodiments of the inventions of the present disclosure as well as various structures and advantages of the inventions of the present disclosure will become further apparent from the following detailed description of various embodiments of the inventions of the present disclosure read in conjunction with the accompanying drawings. The detailed description and drawings are merely illustrative of the inventions of the present disclosure rather than limiting, the scope of the invention of the present disclosure being defined by the appended claims.

### BRIEF DESCRIPTION OF THE DRAWINGS

FIG. 1 illustrates an exemplary embodiment of optimal imaging POV intervention system in accordance with the inventive principles of the present disclosure.
FIGS. 2A-2F illustrate an exemplary optimal imaging POV suggestion in accordance with the inventive principles of the present disclosure.
FIG. 3 illustrates an exemplary embodiment of the optimal imaging POV intervention system of FIG. 1 in accordance with the inventive principles of the present disclosure.
FIG. 4 illustrates an exemplary embodiment of an optimal imaging POV controller in accordance with the inventive principles of the present disclosure.
FIG. 5 illustrates a flowchart representative of an optimal imaging POV intervention method in accordance with the inventive principles of the present disclosure. This method does not form part of the present invention.

### DETAILED DESCRIPTION OF THE PREFERRED EMBODIMENTS

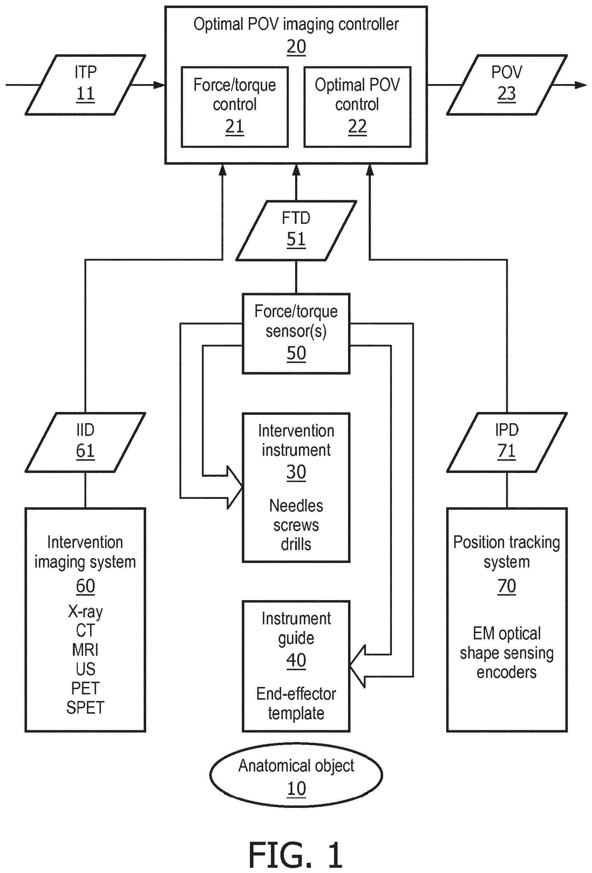

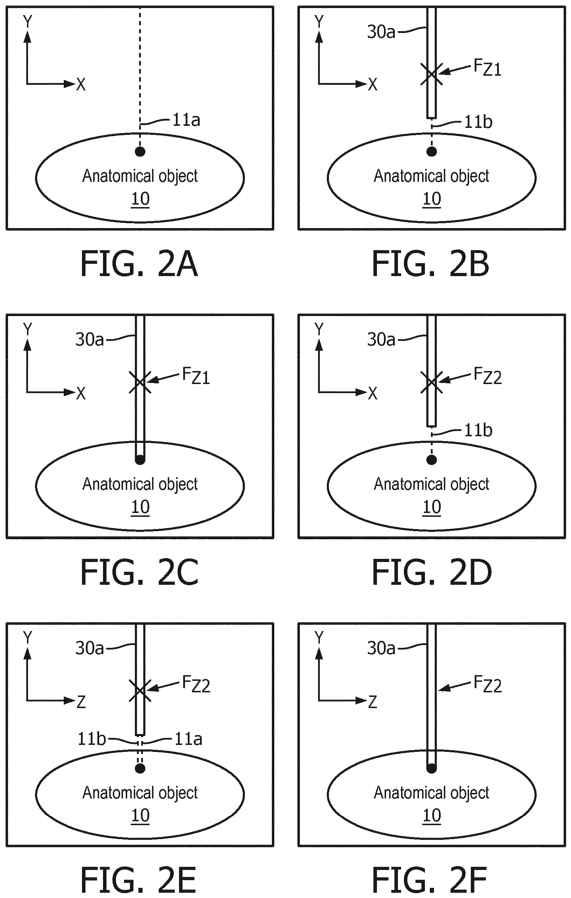

To facilitate an understanding of the various inventions of the present disclosure, the following description of FIGS. 1 and 2 teaches basic inventive principles associated with optimal imaging POV intervention systems of the present disclosure. From this description, those having ordinary skill in the art will appreciate how to apply the inventive principles of the present disclosure for making and using additional embodiments of optimal imaging POV intervention systems the present disclosure.

Referring to FIG. 1, an optimal imaging POV intervention system of the present disclosure employs an intervention instrument 30, an instrument guide 40, an intervention imaging system 60 and a position tracking system 70.

Examples of intervention instrument 30 include, but are not limited to, vascular interventional tools (e.g., guidewires, catheters, stents sheaths, balloons, atherectomy catheters, IVUS imaging probes, deployment systems, etc.), endoluminal interventional tools (e.g., endoscopes, bronchoscopes, etc.) and orthopedic interventional tools (e.g., k-wires and screwdrivers).

In practice, instrument guide 40 may be a stand-alone guide such as, for example, handheld guide devices and templates. Also in practice, instrument guide 40 may be an end-effector of a guide positioning system, such as, for example, an end-effector of a passive robot platform or an end-effector of an active navigation robot. Examples of active navigation robots include, but are not limited to, the da Vinci^{®} Robotic System, the Medrobotics Flex^{®} Robotic System, the Magellan^{™} Robotic System, and the CorePath^{®} Robotic System.

Examples of intervention imaging system 60 include, but are not limited to, a stand-alone x-ray imaging system, a mobile x-ray imaging system, an ultrasound volumetric imaging system (e.g., TEE, TTE, IVUS, ICE), a computed tomography ("CT") imaging system (e.g., a cone beam CT), a positron emission tomography ("PET") imaging system and a magnetic resonance imaging ("MRI") system.

Examples of position tracking system 70 include, but are not limited to, an electromagnetic ("EM") measurement system (e.g., the Auora^{®} electromagnetic measurement system), an optical-fiber based measurement system (e.g., Fiber-Optic RealShape^{™} ("FORS") measurement system), an ultrasound measurement system (e.g., an InSitu or image-based US measurement system), an optical measurement system (e.g., a Polaris optical measurement system), a radio frequency identification measurement system, a magnetic measurement system and an encoder system of active intervention robots.

Generally as known in the art of present disclosure, during an interventional procedure, instrument guide 40, intervention imaging system 60 and position tracking system 70 support an image-guided navigation of intervention instrument 30 relative to an anatomical object 10 in accordance with an intervention treatment plan delineating one or more trajectories for intervention instrument 30 to target location(s) of anatomical object 10.

While position tracking system 70 has proven to very beneficial during the interventional procedures, the invention of the present disclosure address potential positional errors that may arise due to forces/torques exerted on intervention instrument 30 and/or instrument guide 40 that are undetectable from a current imaging POV of intervention imaging system 60 of intervention instrument 30 relative to anatomical object 10. To this end, the optimal imaging POV intervention system of the present disclosure employs an optimal POV imaging controller 20 and one or more force/torque sensor(s) 50 to implement an optimal imaging POV intervention method of the present disclosure.

In practice, force/torque sensor(s) 50 may be embedded in intervention instrument 30 or instrument guide 40, incorporated within a guide positioning system employing instrument guide 40 and/or embodied within a force/torque control module 21 of optimal POV imaging controller 20 (not shown) to measure one or more forces and torques applied, directly and/or indirectly, to intervention instrument 30 and/or instrument guide 40.

In practice, force/torque sensor(s) 50 may have one or more degrees of freedom.

In one embodiment, a force/torque sensor 50 is a six (6) degree of freedom (DOF) (3 axis force/3 axis torque) sensor positioned between the instrument guide 40 and associated guide positioning system. The measured force/torque is gravity compensated for the weight of intervention instrument 30, resolved at the estimated tip position of intervention instrument 30 and transformed into the imaging coordinate system of intervention imaging system 60.

In another embodiment, as known in the art of the present disclosure, a force/torque control module 21 may infer forces/torques exerted on intervention instrument 30 and/or instrument guide 40 from deflections of intervention instrument 30 and/or instrument guide 40, a motor current in a guide positioning system associated with instrument guide 40 or pressure sensing of intervention instrument 30 and/or instrument guide 40.

The optimal imaging POV intervention method involves an inputting of an intervention treatment plan 11 by force/torque control module 21, and a monitoring and storing by force/ torque control module 21 of force/torque data 51, intervention imaging data 61 and intervention position data 71.

Force/torque control module 21 initiates a force/torque analysis when a target position and orientation are within the acceptable threshold (e.g., 1 Degree, 1mm), i.e., intervention instrument 30 is aligned with the target trajectory and moving down the instrument guide 40, or when intervention instrument 30 penetrates skin of the patient (derived from position data, or a force sensor event). Thereafter, force/torque control module 21 signal a surgeon with a beep or graphical warning whenever the force/torque analysis exceeds an empirically predefined threshold.

In practice, the empirically predefined threshold is established a demarcation between positional errors undetected by position tracking system 70 whereby a current navigation trajectory of intervention instrument 30 is within an acceptable tolerance of the target location and between positional errors undetected by position tracking system 70 whereby a current navigation trajectory of intervention instrument 30 is not within an acceptable tolerance of the target location.

In response to the alert, optimal POV control 22 calculates a new imaging axis in the imaging coordinate system of intervention imaging system 60 that is defined by the location of the resolved force/torque, and a vector in the image coordinate system of intervention imaging system 60 formed by a cross product of the target trajectory and the force/torque vector. Thereafter, optimal POV control 22 communicates a new imaging axis (position and the angles) to the surgeon and suggest repositioning intervention imaging system 60 at the new POV to acquire a more optimal view of intervention instrument 30. Alternatively, optical POV control 22 may autonomously control a repositioning of intervention imaging system 60 at the new POV.

For example, FIG. 2A illustrates a planned trajectory 11a in an XY plane imaging POV of anatomical object 10 having a target location symbolized by the black dot in anatomical object 10. As an intervention instrument 30a is navigated toward the target location along a navigation trajectory, force/torque control 21 performs the force/torque analysis in view of the empirically predefined threshold.

When the force/torque analysis is less than the threshold, the navigation of intervention instrument 30a along a navigation trajectory 11b as viewed in the XY plane is uninterrupted by force/torque control 21 to thereby proceed to the target location. FIG. 2B illustrates a force F_{Z1} exerted on intervention instrument 30a that is less than the empirically predefined threshold whereby the navigation of intervention instrument 30a along navigation trajectory 11b as viewed in the XY plane is uninterrupted by force/torque control 21 to thereby proceed to the target location as shown in FIG. 2C. In this case, force Fzi has not caused any significant differential between planned trajectory 11a and navigation trajectory 11b.

When the force/torque analysis exceeds the threshold, the navigation of intervention instrument 30a along navigation trajectory 11b as viewed in the XY plane is interrupted by force/torque control 21 to thereby determine a more optimal imaging POV. FIG. 2D illustrates a force F_{Z2} exerted on intervention instrument 30a that exceeds the empirically predefined threshold whereby the navigation of intervention instrument 30a along navigation trajectory 11b as viewed in the XY plane is interrupted by force/torque control 21 to thereby determine a more optimal imaging POV. For this example, based on the Z-direction of force F_{Z2}, optimal POV control 22 determines a more optimal imaging POV is the ZY plane as shown in FIG. 2E whereby a surgeon or optimal POV control may reposition intervention imaging system 60 accordingly. As a result, the differential between planned trajectory 11a and navigation trajectory 11b enables a surgeon or an autonomous imaging-guided guide positioning system to adjust the navigation of intervention instrument 30 to match planned trajectory 11a to thereby reach the target location as shown in FIG. 2F. In this case, the determination of the optimal imaging POV addressed a significant differential caused by force F_{Z2} between planned trajectory 11a and navigation trajectory 11b that was undetected by position tracking system 70 and not visibly ascertainable in the XY plane imaging POV.

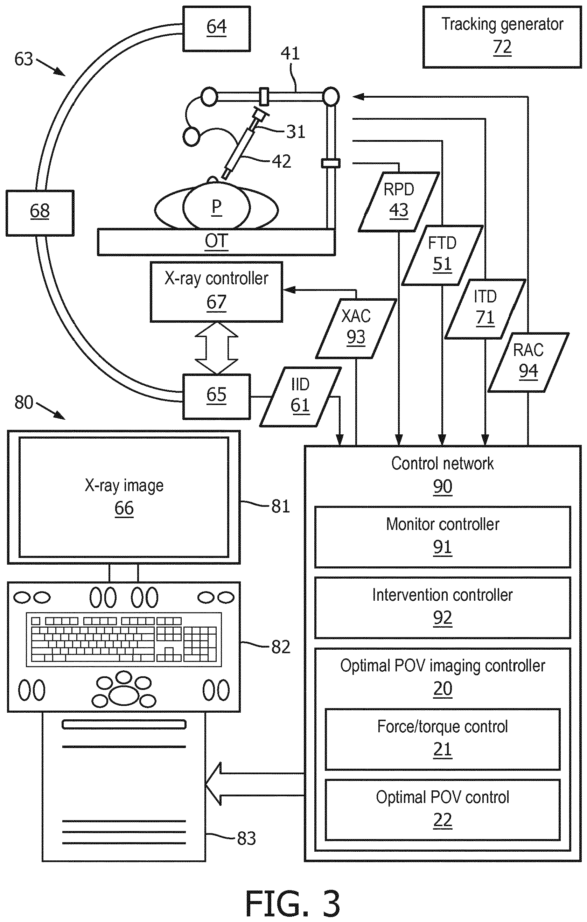

To facilitate a further understanding of the invention of the present disclosure, the following description of FIGS. 3-5 teaches exemplary embodiments of optimal imaging POV intervention systems of the present disclosure From this description, those having ordinary skill in the art will appreciate how to apply the inventive principles of the present disclosure for making and using additional embodiments of optimal imaging POV intervention systems of the present disclosure.

Referring to FIG. 3, an optimal imaging POV system employs a X-ray imaging system 63 (e.g., a mobile c-arm as shown), a robot platform 41 (passive or active), a tracking generator 72 (e.g., an EM generator, an optical generator), an intervention workstation 80 and a control network 90 including a monitor controller 91, an intervention controller 92 and optimal POV imaging controller 20 (FIG. 1) for deploying an interventional instrument 31 held by robot platform 41 within an anatomical region of a patient P lying prone on an operating table OT during an interventional procedure of any type.

As known in the art of the present disclosure, X-ray imaging system 63 generally includes an X-ray generator 64, an image intensifier 65 and a collar 68 for rotating X-ray imaging system 63. In operation as known in the art, an X-ray controller 67 controls a generation by X-ray imaging system 63 of X-ray imaging data 61 informative of a X-ray imaging of the anatomical region of patient P (e.g., a heart of patient P during a minimally invasive aortic valve replacement or vertebrae during a minimally invasive screw placement).

In practice, X-ray controller 67 may be installed within an X-ray imaging workstation (not shown), or alternatively installed within intervention workstation 80.

Still referring to FIG. 3, robot platform 41 provides for a manual or controlled lateral translation and angular movements of end-effector 42. Robot platform 41 may further include an one or more encoders (not shown) and one or more force/torque sensors (not shown).

The encoders are any type of encoder as known in the art of the present disclosure for generating robot pose data 43 informative of a location and/or orientation of each arm/link of robot platform 41 relative to a reference to thereby facilitate a determination by an interventional controller 92 of a pose of intervention instrument 31 as held by robot platform 41 within the anatomical region of patient P.

The force/torque sensors are any type of sensor as known in the art of the present disclosure for generating force torque sensor data 51 informative of a degree of any force applied by robot platform 41 via interventional instrument 31 or robot platform 41 itself to tissue within the anatomical region of patient P. Alternatively, the force/torque sensors may be embedded within intervention instrument 31 or implemented as force/torque algorithms of force/torque control 21 as known in the art of the present disclosure.

Still referring to FIG. 3, intervention workstation 80 is assembled in a known arrangement of a standalone computing system employing a monitor 81, a keyboard 82 and a computer 83.

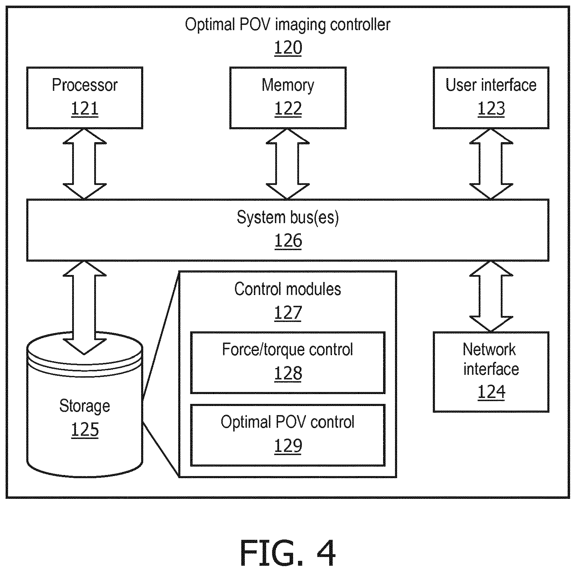

Control network 90 is installed on computer 83. As installed on computer 83, an embodiment 120 of POV imaging controller 20 includes processor(s) 121, memory 122, a user interface 123, a network interface 124, and a storage 125 interconnected via one or more system buses 126 as shown in FIG. 4.

Referring to FIG. 4, each processor 121 may be any hardware device, as known in the art of the present disclosure or hereinafter conceived, capable of executing instructions stored in memory or storage or otherwise processing data. In a non-limiting example, each processor 121 may include a microprocessor, field programmable gate array (FPGA), application-specific integrated circuit (ASIC), or other similar devices.

The memory 122 may include various memories, as known in the art of the present disclosure or hereinafter conceived, including, but not limited to, LI, L2, or L3 cache or system memory. In a non-limiting example, the memory 122 may include static random access memory (SRAM), dynamic RAM (DRAM), flash memory, read only memory (ROM), or other similar memory devices.

The user interface 123 may include one or more devices, as known in the art of the present disclosure or hereinafter conceived, for enabling communication with a user such as an administrator. In a non-limiting example, the user interface 123 may include a command line interface or graphical user interface that may be presented to a remote terminal via the network interface.

The network interface 124 may include one or more devices, as known in the art of the present disclosure or hereinafter conceived, for enabling communication with other hardware devices. In an non-limiting example, the network interface 124 may include a network interface card (NIC) configured to communicate according to the Ethernet protocol. Additionally, the network interface may implement a TCP/IP stack for communication according to the TCP/IP protocols. Various alternative or additional hardware or configurations for the network interface will be apparent\

The storage 125 may include one or more machine-readable storage media, as known in the art of the present disclosure or hereinafter conceived, including, but not limited to, read-only memory (ROM), random-access memory (RAM), magnetic disk storage media, optical storage media, flash-memory devices, or similar storage media. In various non-limiting embodiments, the storage 125 may store instructions for execution by the processor or data upon with the processor may operate. For example, the storage 125 may store a base operating system for controlling various basic operations of the hardware. For purposes of the invention of the present disclosure, storage 125 stores control modules 127 in the form of executable software/firmware for implementing the various functions of force/torque control 128 and optical POV control 129 as further described in the present disclosure.

Referring back to FIG. 3, monitor controller 91 and interventional controller 92 may be segregated, or partially or entirely integrated within optimal PV imaging controller 20. Alternatively, monitor controller 91 and interventional controller 92 may be distributed in any manner between two (2) workstations 80.

In operation, monitor controller 91 processes X-ray image data 61 to generate an X-ray image 66 and controls a display of X-ray image 66 on monitor 81 as known in the art of the present disclosure. Monitor controller 91 further controls a display on monitor 81 of an overlay of planned trajectories and navigated trajectories of intervention instrument 31 as known in the art of the present disclosure.

Intervention controller 92 processes robot position data 35 and intervention position data 71 to control a posing of robot platform 41 to facilitate a navigation of intervention instrument 31 within the anatomical region of patient P.

Force/torque control 21 and optical POV control 22 execute a flowchart 200 representative an optimal imaging POV intervention method of the present disclosure.

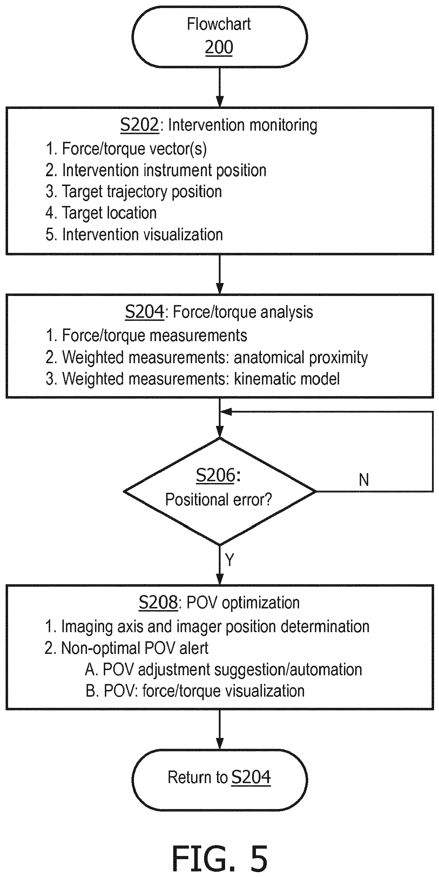

Referring to FIG. 5, a stage S202 of flowchart 200 encompasses force/torque control 21 monitoring and storing force/torque vector(s) as indicated by force/torque data 51 (FIG. 3), an intervention instrument position and target trajectory position as indicated by robot position data 43 (FIG. 3) and intervention tracking data 71 (FIG. 3) and a target location indicated by the planned treatment.

Also during stage S202, force/torque control 21 starts to monitor and store a history of instrument positions whereby a 3D user interface for displaying the history of instrument positions and the forces/torques associated with those positions in the 3D model may be generated during a stage S208 of flowchart 200 in conjunction with any warning of possible positional errors. If generated, then the surgeon may cycle through the history like a VCR movie playback (replay), showing position of intervention instrument 31 and a vector (length showing intensity) of the forces, at a given time in the surgical task. The surgeon may select the scene where he or she believes that the force/torque loading could have affected the procedure whereby this measurement data is then used to suggest an imaging axis.

In addition, the 3D model facilitates a generation of virtual X-ray projections with points of view generated based on the corresponding instrument position and force/torque measurement analysis. The virtual images may be compared to real verification images: side by side or overlaid. In some cases, instead of projections, virtual slices defined by tool position and force direction, and thickness may be presented. Besides the virtual 3D model replay, corresponding operating room video and audio are synchronized (via timestamps) and presented along the force/torque measurements and instrument position data.

Still referring to FIG. 5, a stage S204 of flowchart 200 encompasses force/torque control 21 performing the force/torque analysis.

In practice, force/torque control 21 may measure the force/torque in a variety of modes including, but not limited to measuring a maximum force/torque, measuring force/torque immediately after an event, averaging force/torque for a period when instrument 30 is not moving, and averaging force/torque in a given time period.

Additionally, force/torque control 21 may execute weighted measurements force/torque based on anatomical proximity of intervention instrument 30. More particularly, a number of loading events during a particular instrument insertion into the body may occur. For example, when inserting a needle into the body, the lateral forces maybe initially low going through the skin, and increase when the needle enters the muscle layer because it has more mass to push against. It is then possible to use anatomical information to weigh the importance of the forces/torques based on the location of the instrument relative to the anatomy. For a given measurement set (or in real-time), the forces are weighted by the associated instrument distances relative to the anatomy, and then maximum of these weighted force/torque measurements and corresponding position is used to calculate the imaging axis. As different force/torque thresholds and weights may be used for different instruments, different steps in the procedure and different approach angles.

Furthermore, force/torque control 21 may execute weighted measurements force/torque based on kinematic model of the instrument guide. More particularly, a stiffness of mechanical devices varies with their given configuration and the direction of the loading. If an arm of robot platform 41 is extended, then a given force on the distal end of robot platform 41 will cause a greater torque on the base joint of robot platform 41 than if the arm is retracted. Therefore, the displacement from mechanical (or electrical in case of motors) compliance of the device will be higher at the extend configuration. Similarly, device compliance may be asymmetric. The model of the compliance is used to normalize the force/torque measurements in Cartesian space. For example, if a robot platform 41 was stiffer in Z, than in X direction, the same force magnitude force applied by the tissue in Z and X onto intervention instrument 31 would result in bigger deformation in X with smaller X direction force magnitude.

A stage S206 of flowchart 200 involves a detection of a positional error via a comparison of a force/torque measurement to the threshold. If not, then stage S204 is continually repeated until such time flowchart 200 is terminated upon completion of the procedure or a positional error is detected.

A stage S208 of flowchart 200 encompasses optimal POV control 22 determining an imaging axis and imager position to acquire an optimal POV.

Optimal POV control 22 may calculate a new imaging axis in X-Ray imaging coordinate system that is defined by the location of the force/torque measurement, and a vector in the image coordinate system formed by a cross product of the target trajectory and the force vector.

Where only torque is considered, the axis of the torque and the instrument position is used as the suggested imaging axis.

In cases where force and torque are measured, two or more different imaging axis may be calculated. One way to resolve this is to take an average of these vectors and present that imaging axis to the surgeon. Weighting may also be used.

A maximum of the magnitude between the force and torque may be chosen as the imaging axis based on empirically established torque conversion factor: max_of ( k ^{∗} mag(T) , mag (F) )

Also in practice, instead of using the instrument position, an estimated target position may be utilized by optimal POV control 22 to calculate the imaging axis whereby the force/torque location is translated onto the target position.

The determined imaging axis may be communicated to the surgeon or the X-ray C-arm may be automatically rotated or translated in position after the force event is detected. The X-ray may be automatically triggered to image when it reaches the target position.

In case where the imaging angle or position has areas that are obstructed by opaque objects, an alternative view may be suggested. This view is as close as possible to the normal of the originally suggested viewing axis. This is can be solved using techniques known in the art related to motion planning. Such adjustments are communicated to the surgeon.

Further in practice, a motion constraint may be applied to a robot that aligns possible motion of the instrument to a plane passing through a point where the force event was and parallel to the POV plane. Motion may also be constraint along the force vector.

Upon the adjustment, flowchart 200 returns to stage S204.

In practice, to minimize the possible search space for maximum force/torque loading, the force/torque and positional measurements are tagged (synchronized temporally) with activation of instrumentation such as, for example, an activation/deactivation of the instrument (e.g., bone drill on/off, a detection of a force event through vibrations detected by the sensor (hit bone, hammer) and/or an acoustic sensing of instrument operation. The surgeon is presented with one of the feedback methods mentioned above, or a timeline showing forces and various events, along with a corresponding virtual 3D view of instrument position and force/torque.

Referring to FIGS. 1-5, those having ordinary skill in the art of the present disclosure will appreciate numerous benefits of the invention of the present disclosure including, but not limited to, an ability to adjust imaging POV in view of potential positional errors of an intervention instrument due to forces/torques exerted on the intervention instrument and/or instrument guide.

Further, as one having ordinary skill in the art will appreciate in view of the teachings provided herein, structures, elements, components, etc. described in the present disclosure/specification and/or depicted in the Figures may be implemented in various combinations of hardware and software, and provide functions which may be combined in a single element or multiple elements. For example, the functions of the various structures, elements, components, etc. shown/illustrated/depicted in the Figures can be provided through the use of dedicated hardware as well as hardware capable of executing software in association with appropriate software for added functionality. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, combinations thereof, etc.) which is capable of (and/or configurable) to perform and/or control a process.

Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Having described preferred and exemplary embodiments of novel and inventive image guidance of steerable introducers, and systems incorporating such image guidance of steerable introducers, (which embodiments are intended to be illustrative and not limiting), it is noted that modifications and variations can be made by persons skilled in the art in light of the teachings provided herein, including the Figures. It is therefore to be understood that changes can be made in/to the preferred and exemplary embodiments of the present disclosure which are within the scope of the embodiments disclosed herein.

Moreover, it is contemplated that corresponding and/or related systems incorporating and/or implementing the device/system or such as may be used/implemented in/with a device in accordance with the present disclosure are also contemplated and considered to be within the scope of the present disclosure.

## Claims

1. An optimal imaging POV intervention system, comprising:
an intervention instrument (30);
an instrument guide (40) configured to establish a planned trajectory of the intervention instrument (30);
at least one force/torque sensor (50) configured to sense at least one of a force and a torque exerted against at least one of the intervention instrument (30) and the instrument guide (40) when the intervention instrument (30) is positioned within the instrument guide (40); and
an optimal imaging POV controller (20) operable for controlling a determination of an optimal imaging POV of the intervention instrument (30), wherein the optimal imaging POV controller (20) is configured to:
derive an imaging axis of the intervention instrument (30) from a measurement of at least one of the force and the torque exerted against at least one of the intervention instrument (30) and the instrument guide (40) as sensed by the at least one force/torque sensor (50).

2. The optimal imaging POV intervention system of claim 1, wherein the at least one force/torque sensor (50) is embedded in at least one of the intervention instrument (30) and the instrument guide (40).

3. The optimal imaging POV intervention system of claim 1, further comprising:
a guide positioning system configured to position the instrument guide (40) to establish the planned trajectory of the intervention instrument (30),
wherein the guide positioning system includes the at least one force/torque sensor (50).

4. The optimal imaging POV intervention system of claim 1, where the optimal imaging POV controller (20) includes a force/torque module (21) embodying the at least one force/torque sensor (50), wherein the force/torque module is configured to infer forces/torques exerted on the intervention instrument (30) and/or the instrument guide (40) from (i) deflections of the intervention instrument (30) and/or the instrument guide (40), (ii) a motor current in a guide positioning system associated with the instrument guide (40) or (iii) pressure sensing of the intervention instrument (30) and/or the instrument guide (40).

5. The optimal imaging POV intervention system of claim 1, wherein the optimal imaging POV controller (20) is configured to:
execute at least one of a force measurement and a torque measurement as a maximum of at least one of the force and the torque exerted against at least one of the intervention instrument (30) and the instrument guide (40).

6. The optimal imaging POV intervention system of claim 1, wherein the optimal imaging POV controller (20) is configured to:
execute at least one of a force measurement and a torque measurement as at least one of the force and the torque exerted against at least one of the intervention instrument (30) and the instrument guide (40) as sensed after an event.

7. The optimal imaging POV intervention system of claim 1, wherein the optimal imaging POV controller (20) is configured to:
execute at least one of a force measurement and a torque measurement as an average of at least one of the force and the torque exerted against at least one of the intervention instrument (30) and the instrument guide (40) over a period of time.

8. The optimal imaging POV intervention system of claim 1, wherein the optimal imaging POV controller (20) is configured to:
execute at least one of a force measurement and a torque measurement as an average of at least one of the force and the torque exerted against at least one of the intervention instrument (30) and the instrument guide (40) when the intervention instrument (30) is stationary within the instrument guide (40).

9. The optimal imaging POV intervention system of claim 1, wherein the optimal imaging POV controller (20) is configured to:
execute at least one of a weighted force measurement and a weighted torque measurement of at least one of the force and the torque exerted against at least one of the intervention instrument (30) and the instrument guide (40) based on a proximity of the intervention instrument (30) to a target location of the planned trajectory.

10. The optimal imaging POV intervention system of claim 1, wherein the optimal imaging POV controller (20) is configured to:
execute at least one of a weighted force measurement and a weighted torque measurement of at least one of the force and the torque exerted against at least one of the intervention instrument (30) and the instrument guide (40) based on a kinematic model of the instrument guide (40).

11. The optimal imaging POV intervention system of claim 1, wherein the optimal imaging POV controller (20) is configured to:
calculate the imaging axis as function of a location of a force/torque vector exerted against at least one of the intervention instrument (30) and the instrument guide (40) as sensed by the at least one force/torque sensor (50) and an imaging vector in an imaging coordinate system formed by a cross product of the planned trajectory and the force/torque vector.

12. The optimal imaging POV intervention system of claim 1, wherein the optimal imaging POV controller (20) is configured to:
calculate the imaging axis as an average of a force vector exerted against the at least one of the intervention instrument (30) and the instrument guide (40) as sensed by the at least one force/torque sensor (50) and a torque vector exerted against at least one of the intervention instrument (30) and the instrument guide (40) as sensed by the at least one force/torque sensor (50).

13. The optimal imaging POV intervention system of claim 1, wherein the optimal imaging POV controller (20) is configured to at least one of:
control a communication of the imaging axis to an intervention imaging system; and
control a repositioning of an intervention imaging system in accordance with the imaging axis.

14. The optimal imaging POV intervention system of claim 1, wherein the optimal imaging POV controller (20) is configured to at least one of:
control a visualization of a model illustrative of positions of the intervention instrument (30) and measurements of at least one of a force and a torque associated with the positions of the intervention instrument (30) relative to the planned trajectory; and
control a visualization of a model illustrative of a plurality of imaging POVs of intervention instrument (30) and measurements of at least one of a force and a torque associated with the positions of the intervention instrument (30) relative to the planned trajectory.

15. The optimal imaging POV intervention system of claim 1, wherein the optimal imaging POV controller (20) is configured to at least one of:
control a motion of an image guidance system to align motion of the intervention instrument (30) a plane passing through a point at force event sensed by the at least one force/torque sensor (50) and parallel to a POV plane; and
control a motion of an image guidance system along a force vector sensed by the at least one force/torque sensor.

## Patentansprüche

1. Ein Eingriffssystem mit optimaler POV-Bildgebung, das Folgendes umfasst:
ein Eingriffsinstrument (30);
eine Instrumentenführung (40), die die geplante Bahn des Eingriffsinstruments (30) einrichtet;
mindestens einen Kraft-/Drehmomentsensor (50), der mindestens eine Kraft und/oder ein Drehmoment erfasst, die/das auf das Eingriffsinstrument (30) und/oder die Instrumentenführung (40) ausgeübt werden, wenn sich das Eingriffsinstrument (30) in der Instrumentenführung (40) befindet; und
eine Steuerung für die optimale POV-Bildgebung (20) zum Steuern des Erkennens einer optimalen POV-Bildgebung für das Eingriffsinstrument (30), wobei die Steuerung für eine optimale POV-Bildgebung (20) folgenden Schritt durchführt:
Ableiten der Bildgebungsachse des Eingriffsinstruments (30) aus einer mit dem mindestens einen Kraft/Drehmomentsensor (50) durchgeführten Messung der Kraft und/oder des Drehmoments, die/das auf das Eingriffsinstrument (30) und/oder die Instrumentenführung (40) ausgeübt wird.

2. Das Eingriffssystem mit optimaler POV-Bildgebung gemäß Anspruch 1, wobei der mindestens eine Kraft-/Drehmomentsensor (50) in mindestens das Eingriffsinstrument (30) und die Instrumentenführung (40) integriert ist.

3. Das Eingriffssystem mit optimaler POV-Bildgebung gemäß Anspruch 1, das zudem Folgendes umfasst:
ein Führungspositionierungssystem, das die Instrumentenführung (40) positioniert, um die geplante Bahn des Eingriffsinstruments (30) einzurichten,
wobei das Führungspositionierungssystem den mindestens einen Kraft-/Drehmomentsensor (50) umfasst.

4. Das Eingriffssystem mit optimaler POV-Bildgebung gemäß Anspruch 1, wobei die Steuerung für die optimale POV-Bildgebung (20) ein Kraft-/Drehmomentmodul (21) umfasst, das den mindestens einen Kraft-/Drehmomentsensor (50) umfasst, wobei das Kraft-/Drehmomentmodul die auf das Eingriffsinstrument (30) und/oder die Instrumentenführung (40) ausgeübten Kräfte/Drehmomente ableitet aus (i) Auslenkungen des Eingriffsinstruments (30) und/oder der Instrumentenführung (40), (ii) dem Motorstrom eines mit der Instrumentenführung (40) verbundenen Führungspositionierungssystems oder (iii) einer Druckmessung für das Eingriffsinstrument (30) und/oder die Instrumentenführung (40).

5. Das Eingriffssystem mit optimaler POV-Bildgebung gemäß Anspruch 1, wobei die Steuerung für eine optimale POV-Bildgebung (20) folgenden Schritt durchführt:
Ausführen mindestens einer Kraftmessung und/oder Drehmomentmessung als Höchstwert der Kraft und/oder des Drehmoments, die/das auf das Eingriffsinstrument (30) und/oder die Instrumentenführung (40) ausgeübt wird.

6. Das Eingriffssystem mit optimaler POV-Bildgebung gemäß Anspruch 1, wobei die Steuerung für eine optimale POV-Bildgebung (20) folgenden Schritt durchführt:
nach einem Ereignis Ausführen mindestens einer Kraftmessung und/oder Drehmomentmessung der Kraft und/oder des Drehmoments, die/das auf das Eingriffsinstrument (30) und/oder die Instrumentenführung (40) ausgeübt wird.

7. Das Eingriffssystem mit optimaler POV-Bildgebung gemäß Anspruch 1, wobei die Steuerung für eine optimale POV-Bildgebung (20) folgenden Schritt durchführt:
Ausführen mindestens einer Kraftmessung und/oder Drehmomentmessung als Mittelwert der Kraft und/oder des Drehmoments, die/das auf das Eingriffsinstrument (30) und/oder die Instrumentenführung (40) ausgeübt wird.

8. Das Eingriffssystem mit optimaler POV-Bildgebung gemäß Anspruch 1, wobei die Steuerung für eine optimale POV-Bildgebung (20) folgenden Schritt durchführt:
Ausführen einer Kraftmessung und/oder einer Drehmomentmessung als Mittelwert der Kraft und/oder des Drehmoments, die/das auf das Eingriffsinstrument (30) und/oder die Instrumentenführung (40) ausgeübt wird,
wenn sich das Eingriffsinstrument (30) stationär in der Instrumentenführung (40) befindet.

9. Das Eingriffssystem mit optimaler POV-Bildgebung gemäß Anspruch 1, wobei die Steuerung für eine optimale POV-Bildgebung (20) folgenden Schritt durchführt:
beruhend auf der Nähe des Eingriffsinstruments (30) zur Zielposition der geplanten Bahn Ausführen einer gewichteten Kraftmessung und/oder Drehmomentmessung der Kraft und/oder des Drehmoments, die/das auf das Eingriffsinstrument (30) und/oder die Instrumentenführung (40) ausgeübt wird.

10. Das Eingriffssystem mit optimaler POV-Bildgebung gemäß Anspruch 1, wobei die Steuerung für eine optimale POV-Bildgebung (20) folgenden Schritt durchführt:
beruhend auf einem kinematischen Modell der Instrumentenführung (40) Ausführen einer gewichteten Kraftmessung und/oder Drehmomentmessung der Kraft und/oder des Drehmoments, die/das auf das Eingriffsinstrument (30) und/oder die Instrumentenführung (40) ausgeübt wird.

11. Das Eingriffssystem mit optimaler POV-Bildgebung gemäß Anspruch 1, wobei die Steuerung für eine optimale POV-Bildgebung (20) folgenden Schritt durchführt:
Berechnen der Bildgebungsachse als Funktion einer Stelle eines vom mindestens einen Kraft-/Drehmomentsensor (50) erfassten Kraft-/Drehmomentvektors, der auf das Eingriffsinstrument (30) und/oder die Instrumentenführung (40) ausgeübt wird, sowie eines Bildgebungsvektors in einem Bildgebungskoordinatensystem, der durch das Vektorprodukt der geplanten Bahn und dem Kraft-/Drehmomentvektor gebildet wird.

12. Das Eingriffssystem mit optimaler POV-Bildgebung gemäß Anspruch 1, wobei die Steuerung für eine optimale POV-Bildgebung (20) folgenden Schritt durchführt:
Berechnen der Bildgebungsachse als Mittelwert eines vom mindestens einen Kraft-/Drehmomentsensor (50) erfassten Kraftvektors, der auf das Eingriffsinstrument (30) und/oder die Instrumentenführung (40) ausgeübt wird, sowie eines vom mindestens einen Kraft-/Drehmomentsensor (50) erfassten Drehmomentvektors,
der auf das Eingriffsinstrument (30) und/oder die Instrumentenführung (40) ausgeübt wird.

13. Das Eingriffssystem mit optimaler POV-Bildgebung gemäß Anspruch 1, wobei die Steuerung für eine optimale POV-Bildgebung (20) mindestens einen der folgenden Schritte durchführt:
Steuern der Kommunikation der Bildgebungsachse mit einem Eingriffsbildgebungssystem; und
Steuern der Neupositionierung eines Eingriffsbildgebungssystems anhand der Bildgebungsachse.

14. Das Eingriffssystem mit optimaler POV-Bildgebung gemäß Anspruch 1, wobei die Steuerung für eine optimale POV-Bildgebung (20) mindestens einen der folgenden Schritte durchführt:
Steuern der Visualisierung eines Modells, das das Positionen des Eingriffsinstruments (30) veranschaulicht, sowie Messen einer Kraft und/oder eines Drehmoments, die/das den Positionen des Eingriffsinstruments (30) relativ zur geplanten Bahn zugeordnet ist;
und
Steuern der Visualisierung eines Modells, das mehrere Bilgebungs-POVs des Eingriffsinstruments (30) veranschaulicht, sowie Messen einer Kraft und/oder eines Drehmoments, die/das den Positionen des Eingriffsinstruments (30) relativ zur geplanten Bahn zugeordnet ist.

15. Das Eingriffssystem mit optimaler POV-Bildgebung gemäß Anspruch 1, wobei die Steuerung für eine optimale POV-Bildgebung (20) mindestens einen der folgenden Schritte durchführt:
Steuern der Bewegung eines Bildführungssystems, um die Bewegung des Eingriffsinstruments (30) auf eine Ebene auszurichten, die durch einen Punkt eines vom mindestens einen Kraft-/Drehmomentsensor (50) erfassten Kraftereignisses sowie parallel zur POV-Ebene verläuft; und
Steuern der Bewegung eines Bildführungssystems entlang eines vom mindestens einen Kraft-/Drehmomentsensor erfassten Kraftvektors.

## Revendications

1. Système d'intervention sur PDV d'imagerie optimal, comprenant :
un instrument d'intervention (30) ;
un guide d'instrument (40) configuré pour établir une trajectoire planifiée de l'instrument d'intervention (30) ;
au moins un capteur de force/couple (50) configuré pour détecter au moins une force et/ou un couple exercé contre au moins l'instrument d'intervention (30) et le guide d'instrument (40) lorsque l'instrument d'intervention (30) est placé à l'intérieur du guide d'instrument (40) ; et
un dispositif de commande de PDV d'imagerie optimal (20) pour commander une détermination d'un PDV d'imagerie optimal de l'instrument d'intervention (30), le dispositif de commande de PDV d'imagerie optimal (20) étant configuré pour :
dériver un axe d'imagerie de l'instrument d'intervention (30) à partir d'au moins une mesure de la force et du couple exercé contre au moins l'instrument d'intervention (30) et le guide d'instrument (40) tel que détecté par au moins le capteur de force/couple (50).

2. Système d'intervention sur PDV d'imagerie optimal selon la revendication 1, dans lequel le ou les capteurs de force/couple (50) est incorporé dans au moins l'instrument d'intervention (30) et le guide d'instrument (40).

3. Système d'intervention sur PDV d'imagerie optimal selon la revendication 1, comprenant en outre :
un système de placement du guide configuré pour placer le guide d'instrument (40) pour établir la trajectoire planifiée de l'instrument d'intervention (30),
dans lequel le système de placement du guide comprend au moins le capteur de force/couple (50).

4. Système d'intervention sur PDV d'imagerie optimal selon la revendication 1, dans lequel le dispositif de commande de PDV d'imagerie optimal (20) comprend un module de force/couple (21) incarnant au moins le capteur de force/couple (50), le module de force/couple étant configuré pour déduire les forces/couples exercés sur l'instrument d'intervention (30) et/ou le guide d'instrument (40) des (i) déformations de l'instrument d'intervention (30) et/ou du guide d'instrument (40) (ii) un courant moteur dans un système de placement de guide associé au guide d'instrument (40) ou (iii) une détection de pression de l'instrument d'intervention (30) et/ou le guide d'instrument (40).

5. Système d'intervention sur PDV d'imagerie optimal selon la revendication 1, dans lequel le dispositif de commande de PDV d'imagerie optimal (20) est configuré pour :
exécuter au moins une mesure de force et une mesure de couple comme un maximum d'au moins une force et un couple exercé contre au moins l'instrument d'intervention (30) et le guide d'instrument (40).

6. Système d'intervention sur PDV d'imagerie optimal selon la revendication 1, dans lequel le dispositif de commande de PDV d'imagerie optimal (20) est configuré pour :
exécuter au moins une mesure de force et une mesure de couple comme au moins une force et un couple exercé contre au moins l'instrument d'intervention (30) et le guide d'instrument (40) tel que détecté après un événement.

7. Système d'intervention sur PDV d'imagerie optimal selon la revendication 1, dans lequel le dispositif de commande de PDV d'imagerie optimal (20) est configuré pour :
exécuter au moins une mesure de force et une mesure de couple comme une moyenne d'au moins une force et
un couple exercé contre au moins l'instrument d'intervention (30) et le guide d'instrument (40) sur une période de temps.

8. Système d'intervention sur PDV d'imagerie optimal selon la revendication 1, dans lequel le dispositif de commande de PDV d'imagerie optimal (20) est configuré pour :
exécuter au moins une mesure de force et une mesure de couple comme au moins une force et un couple exercé contre au moins l'instrument d'intervention (30) et le guide d'instrument (40) lorsque l'instrument d'intervention (30) est fixe à l'intérieur du guide d'instrument (40).

9. Système d'intervention sur PDV d'imagerie optimal selon la revendication 1, dans lequel le dispositif de commande de PDV d'imagerie optimal (20) est configuré pour :
exécuter au moins une mesure de force pondérée et une mesure de couple pondérée d'au moins une force et un couple exercé contre au moins l'instrument d'intervention (30) et le guide d'instrument (40) sur la base d'une proximité de l'instrument d'intervention (30) à un emplacement cible de la trajectoire planifiée.

10. Système d'intervention sur PDV d'imagerie optimal selon la revendication 1, dans lequel le dispositif de commande de PDV d'imagerie optimal (20) est configuré pour :
exécuter au moins une mesure de force pondérée et une mesure de couple pondérée d'au moins une force et un couple exercé contre au moins l'instrument d'intervention (30) et le guide d'instrument (40) sur la base d'un modèle cinématique du guide d'instrument (40) .

11. Système d'intervention sur PDV d'imagerie optimal selon la revendication 1, dans lequel le dispositif de commande de PDV d'imagerie optimal (20) est configuré pour :
calculer l'axe d'imagerie comme une fonction d'un emplacement d'un vecteur de force/couple exercé contre au moins l'instrument d'intervention (30) et le guide d'instrument (40) tel que détecté par au moins le capteur de force/couple (50) et un vecteur d'imagerie dans un système de coordonnées d'imagerie formé par un produit croisé de la trajectoire planifiée et le vecteur de force/couple.

12. Système d'intervention sur PDV d'imagerie optimal selon la revendication 1, dans lequel le dispositif de commande de PDV d'imagerie optimal (20) est configuré pour :
calculer l'axe d'imagerie comme une moyenne de vecteur de force exercé contre au moins l'instrument d'intervention (30) et le guide d'instrument (40) tel que détecté par au moins le capteur de force/couple (50) et un vecteur de couple exercé contre au moins l'instrument d'intervention (30) et le guide d'instrument (40) tel que détecté par au moins le capteur de force/couple (50).

13. Système d'intervention sur PDV d'imagerie optimal selon la revendication 1, dans lequel le dispositif de commande de PDV d'imagerie optimal (20) est configuré pour au moins :
contrôler une communication de l'axe d'imagerie au système d'imagerie d'intervention ; et
contrôler un replacement d'un système d'imagerie d'intervention selon l'axe d'imagerie.

14. Système d'intervention sur PDV d'imagerie optimal selon la revendication 1, dans lequel le dispositif de commande de PDV d'imagerie optimal (20) est configuré pour au moins :
contrôler une visualisation d'un modèle illustratif des positions de l'instrument d'intervention (30) et les mesures d'au moins une force et un couple associés aux positions de l'instrument d'intervention (30) par rapport à la trajectoire planifiée ; et contrôler une visualisation d'un modèle illustratif d'une pluralité de PDV d'imagerie d'instrument d'intervention (30) et les mesures d'au moins une force et un couple associé aux positions de l'instrument d'intervention (30) par rapport à la trajectoire planifiée.

15. Système d'intervention sur PDV d'imagerie optimal selon la revendication 1, dans lequel le dispositif de commande de PDV d'imagerie optimal (20) est configuré pour au moins :
contrôler un mouvement d'un système de guidage d'image pour aligner le mouvement de l'instrument d'intervention (30) un plan passant par un point à l'événement de force détecté par au moins un capteur de force/couple (50) et parallèle à un plan PDV ; et
contrôler un mouvement d'un système de guidage d'image le long d'un vecteur de force détecté par au moins un capteur de force/couple.
